# EUROPEAN PATENT SPECIFICATION

(11) **EP 4 027 791 B1**
(45) Date of publication and mention of the grant of the patent: **04.10.2023**
(21) Application number: 20772201.8
(22) Date of filing: 26.08.2020
(51) Int. Cl.: A01N 43/76, A01P 1/00

(54) **MICROBIOCIDAL USE**
MIKROBIZIDE VERWENDUNG
UTILISATION ANTIMIROBIENNE

(30) Priority: 11.09.2019 EP 19196624
(43) Date of publication of application: 20.07.2022
(73) Proprietor: DSM IP Assets B.V., 6411 TE Heerlen (NL)
(72) Inventor: MENDROK-EDINGER, Christine, 4303 Kaiseraugst (CH)
(74) Representative: Berg, Katja
(86) International application number: PCT/EP2020/073788
(87) International publication number: WO 2021/047904

(56) References cited:
- WO-A1-02/39972
- WO-A1-2016/077857

## Description

The present invention relates to the non-therapeutic use of 1,4-di(benzoxazol-2'-yl)benzene as antimicrobial agent.

To protect cosmetic or pharmaceutical compositions, household products, plastics, paper and/ or paints against mold and bacteria, most products currently on the market contain preservatives. While these preservatives protect against bacteria and fungi, studies have linked daily exposure to many of these substances to an increased risk of skin irritation, cancer and/ or endocrine problems. Thus, many manufactures are searching for alternative antimicrobial actives which allow reducing the amount of preservatives and don't appear to pose any health risks.

Antimicrobial active compounds furthermore play a key role for many cosmetic applications. They are, for example, added into deodorants to reduce body odor caused by bacterial growth such as by Staphylococcus or to balance the microbiome by suppressing the growth of unwanted bacteria on skin such as Staphylococcus aureus, Escherichia coli and/ or Pseudomonas aeruginosa.

Surprisingly, it has now been found that 1,4-di(benzoxazol-2'-yl)benzene exhibits an antimicrobial activity against a variety of microbes such as in particular Candida albicans (C. albicans), Escherichia coli (E. coli), Staphylococcus aureus (S. aureus), Pseudomonas aeruginosa (P. aeruginosa) and/ or Aspergillus brasiliensis (A. brasiliensis).

Thus, the present invention relates to the use of 1,4-di(benzoxazol-2'-yl)benzene as antimicrobial agent, i.e. an agent which exhibits an antimicrobial activity. In particular the present invention is directed to the use of 1,4-di(benzoxazol-2'-yl)benzene as anti-fungal and/ or anti-bacterial agent, more in particular as an agent for killing and/ or inhibiting the growth of fungi and/ or gram-positive bacteria such as in particular C. albicans, E. coli, S. aureus, P. aeruginosa and/ or A. brasiliensis.

In another embodiment, the invention relates to a method for killing and/ or inhibiting growth of microbial cells, in particular fungal and/ or bacterial cells, said method comprising contacting said microbial cells with 1,4-di(benzoxazol-2'-yl)benzene. In a preferred embodiment, the microbial cells are selected from the group consisting of fungi and/or gram-positive bacteria, more preferably from the group consisting of C. albicans, E. coli, S. aureus, P. aeruginosa and/ or A. brasiliensis as well as mixtures thereof.

The term "antimicrobial activity" (or "antimicrobial effect") as used herein means a capability of killing and/or inhibiting the growth of microbial cells such as in particular bacteria and fungi and more in particular C. albicans, E. coli, S. aureus, P. aeruginosa and A. brasiliensis as well as mixtures thereof.

1,4-Di(benzoxazol-2'-yl)benzene [CAS 904-39-2] is a colorless to light yellow, solid, which can be prepared as e.g. outlined in WO200239972 or illustrated in the example.

Due to its low solubility, in all embodiments of the present invention, the 1,4-di(benzoxazol-2'-yl)benzene is preferably used in micronized form, i.e. in the form of solid particles having a mean particle size of less than 1µm.

In a particular advantageous embodiment, said micronized form exhibits a mean particle size of at most 300 nm. Even more preferably, the micronized 1,4-di(benzoxazol-2'-yl)benzene exhibits a mean particle size selected in the range from 50 to 300 nm, more preferably in the range from 100 to 300 nm, most preferably in the range from 120 to 280 nm, such as in the range from 140 to 240 nm or in the range from 150 to 220 nm.

The term 'mean particle size' as used herein refers to the mean number-based particle size distribution Dₙ50 (also known as Dₙ0.5) as determined by laser diffraction e.g. with a Malvern Mastersizer 2000 (ISO 13320:2009).

The micronized 1,4-di(benzoxazol-2'-yl)benzene according to the present invention can be produced by standard micronization methods in the art as e.g. outlined in WO9522959 and WO9703643 which are included herein by reference.

The micronized 1,4-di(benzoxazol-2'-yl)benzene according to the present invention may be used in powder form or as a dispersion thereof.

The micronized 1,4-di(benzoxazol-2'-yl)benzene according to the present invention can furthermore be in a crystalline or in a solid amorphous form. In all embodiments of the present invention it is preferred, that the micronized 1,4-di(benzoxazol-2'-yl)benzene is in a solid amorphous form.

The term "solid amorphous form," as used herein, refers to solid particles which are formed by fast formation/separation of a solid phase from a liquid phase in a solution or a mixture, so that the solid has no time to selectively form a crystal network and thus the obtained solid is in a predominantly disordered form (also referred to herein as 'solid amorphous 1,4-di(benzoxazol-2'-yl)benzene'), which form can be unambiguously identified by XRPD analysis by not exhibiting a base line separation of the peaks at 25-28 °2Theta (Cu K-alpha Radiation) measured using a Bruker D8 Advance powder X-ray diffractometer in reflection (Bragg-Brentano) geometry with a LynxEye detector and Cu Kα radiation.

The solid amorphous form of 1,4-di(benzoxazol-2'-yl)benzene according to the present invention is preferably prepared by a process encompassing the steps of (1) reacting terephthalic acid and 2-aminophenol to form 1,4-di(benzoxazol-2'-yl)benzene and (2) precipitating the thus obtained 1,4-di(benzoxazol-2'-yl)benzene, preferably in the presence of (ice) water to obtain solid amorphous 1,4-di(benzoxazol-2'-yl)benzene, which, in a consecutive step, is micronized according to standard methods in the art. Optionally, the process can contain intermediate purification steps. Alternatively the solid amorphous 1,4-di(benzoxazol-2'-yl)benzene can be further purified by washing with a solvent, in which the 1,4-di(benzoxazol-2'-yl)benzene is only hardly or not soluble such as e.g. toluene and/ or an alcohol such as 1-butanol without being limited thereto.

It is furthermore advantageous that, in all embodiments of the present invention, the micronized 1,4-di(benzoxazol-2'-yl)benzene is used in form of an aqueous dispersion thereof as this eases the incorporation into the desired product form such as into cosmetic or pharmaceutical compositions.

The amount of the 1,4-di(benzoxazol-2'-yl)benzene in such aqueous dispersions is preferably selected in the range from 10 to 90 wt.-%, 20 to 80 wt.-% or 30 to 70 wt-%, more preferably in the range from 25 to 60 wt.-%, most preferably in the range from 25 to 55 wt.-%, such as in the range from 25 to 50 wt.-%, in the range from 25 to 40 wt.-% or in the range from 25 to 35 wt.-%, based on the total weight of the aqueous dispersion.

The aqueous dispersion may furthermore contain one or more additives e.g. to facilitate micronization and/ or to stabilize the aqueous dispersion e.g. against sedimentation.

If present, the amount (total) of the one or more additives in the aqueous dispersion is preferably selected in the range from 0.01 to 25 wt.-%, more preferably in the range from 0.1 to 20 wt.-%, most preferably in the range from 0.5 to 15 wt.-%, such as in the range from 1 to 10 wt.-%, based on the total weight of the aqueous dispersion.

In all embodiments according to the present invention, the at least one additive is preferably selected from the group of surface-active ingredients such as in particular anionic, non-ionic, amphoteric and cationic surfactants, anti-foam agents, salts, wetting agents and thickeners as well as mixtures thereof.

Particularly suitable surface-active ingredients to be used in the aqueous dispersions are alkyl polyglucosides (APG's) having the generic formula CₙH₂₊ₙ O(C₆H₁₀O₅)ₓH, in which n is an integer selected in the range from 2 to 22 and x refers to the mean polymerization level of the glucoside moiety (mono-, di-, tri-, oligo-, and poly-glucoside). Such alkyl polyglucosides are e.g. commercially available under the tradename Green APG 0810 by Shanghai Fine Chemical (a C₈₋₁₀ alkyl poly-glucoside which is made from glucose derived from corn and C₈ and C₁₀ fatty alcohols derived from coconut and palm kernel oils) or Plantacare 1200 UP from BASF (a C12-C16 fatty alcohol polyglycoside). If present, the surface-active ingredient(s) are preferably used in an amount (total) selected in the range from 1 to 20 wt.-%, more preferably from 5 to 15 wt.-%, most preferably from 7 to 12.5 wt.-%, based on the total weight of the aqueous dispersion.

Particularly suitable anti-foam agents to be used in the aqueous dispersions are silicone oils such as in particular polydimethylsiloxanes and/ or silicon anti-foam agents such as in particular anhydrous dispersions of pyrogenic or hydrophobized silica in silicone oils. Most preferably the anti-foam agent is simethicone. If present, such anti-foam agent(s) are preferably used in an amount (total) selected in the range from 0 to 1 wt.-%, more preferably in an amount of 0.01 to 0.2 wt.-%, based on the total weight of the aqueous dispersion.

Suitable salts to be used in the aqueous dispersions include alkali and earth alkaline salts of phosphate, hydroxide such as e.g. disodium hydrogen phosphate and/ or sodium hydroxide. If present, the salt or mixtures thereof (total amount) are preferably used in amounts from 0.01 to 5 wt.-, more preferably from 0.1 to 4 wt.-%, most preferably from 0.5 to 2.5 wt.-%, based on the total weight of the aqueous dispersion.

Particularly suitable thickeners to be used in the aqueous dispersions encompass xanthan gum, gellan gum and/ or carboxymethylcellulose. Most preferably the thickener is xanthan gum or gellan gum. If present, such thickener(s) are preferably used in an amount (total) selected in the range from 0.1 to 1 wt.-%, more preferably in the range from 0.1 to 0.5 wt.-%, based on the total weight of the aqueous dispersion.

Particularly suitable wetting agents to be used in the aqueous dispersions are (poly)propyleneglycol such as most preferably propyleneglycol. If present, the amount of such wetting agent(s) is preferably selected in the range from 0.1 to 1 wt.-%, more preferably in an amount of 0.2 to 0.6 wt.-%, based on the total weight of the aqueous dispersion.

In all embodiments according to the present invention preferably an aqueous dispersion consisting essentially of the micronized 1,4-di(benzoxazol-2'-yl)benzene, water and optionally at least one additive with all the definitions and preferences as given herein is used.

Even more advantageously, the aqueous dispersions consist essentially of
(i) 25-60 wt.-%, preferably 25-50 wt.-%, based on the total weight of the aqueous dispersion, of the micronized 1,4-di(benzoxazol-2'-yl)benzene with all the preferences and definitions as given herein,
(ii) 5 to 15 wt.-%, preferably 7 to 12.5 wt.-%, based on the total weight of the aqueous dispersion, of a C₈₋₁₆ alkyl polyglucoside,
(iii) 0 to 3 wt.-%, preferably 0 to 2.5 wt.-%, based on the total weight of the aqueous dispersion of at least one additive selected from the group of thickeners, wetting agents and/ or anti-foam agents, and
(iv) water Ad 100 wt.-%, based on the total weight of the aqueous dispersion.

The term 'consists essentially of' as used according to the present invention means that the amounts of the ingredients (i) to (iv) sum up to 100 wt.-%, which is adjusted with the amount of water (iv). It is, however, not excluded that small amount of impurities or additives may be present which are, for example, introduced via the respective raw materials of the ingredients.

Most advantageously, the aqueous dispersions contain as additives (iii) propyleneglycol, one thickener selected from xanthan gum or gellan gum and optionally simethicone.

Due to its antimicrobial activity, 1,4-di(benzoxazol-2'-yl)benzene is also suitable to maintain skin homeostasis and/ or balance the skin microbiome by treating overpopulation of microorganisms on the skin such as e.g. E. coli, S. aureus and/ or P. aerugionosa.

To make use of the anti-microbial activity of 1,4-di(benzoxazol-2'-yl)benzene, it can be incorporated into a multiplicity of formulations or applications, such as, for example, into cosmetic or pharmaceutical compositions, medicinal products, household products, plastics, plastisols, paper and/or paints.

The amount of 1,4-di(benzoxazol-2'-yl)benzene employed according to the present invention is preferably selected in the range from 0.1 to 20 wt.-%, more preferably in the range from about 0.25 to 15 wt.-%, most preferably in the range from about 0.5 to 10 wt.-%, such as in the range from 0.3 to 7.5 wt.-% (always based on the active). Further suitable ranges encompass 0.25 to 10 wt.-%, 0.25 to 7.5 wt.-%, 0.25 to 5 wt.-%, 1 to 7.5 wt.-% as well as 1 to 5 wt.-%.

In a particular embodiment, the present invention relates also to the use of 1,4-di(benzoxazol-2'-yl)benzene for improving preservation, in particular of a product selected from the group of cosmetic and/ or pharmaceutical compositions, household products, plastics, paper and/ or paints, preferably in view of Aspergillus brasiliensis.

The use optionally encompasses the step of appreciating the effect compared to a respective product not containing1,4-di(benzoxazol-2'-yl)benzene.

In another embodiment, the invention relates to a method of preventing microbial decay and breakdown of a product selected from the group of cosmetic and/ or pharmaceutical compositions, household products, plastics, paper and/ or paints, wherein said method comprises adding to the product 1,4-di(benzoxazol-2'-yl)benzene in an amount of 0.1 to 20 wt.-%, more preferably in an amount of 0.25 to 15 wt.-%, most preferably in an amount of 0.3 to 10 wt.-%, such as in an amount of 0.3 to 7.5 wt.-%, based on the total weight of the product. In a particular embodiment, the method also encompasses the step of appreciating the result, in particular in comparison to the respective product not containing 1,4-di(benzoxazol-2'-yl)benzene. Further suitable ranges encompass 0.25 to 10 wt.-%, 0.25 to 7.5 wt.-%, 0.25 to 5 wt.-%, 1 to 7.5 wt.-% as well as 1 to 5 wt.-%.

In a further embodiment, the invention relates to a method of preserving a product selected from the group of cosmetic and/ or pharmaceutical compositions, household products, plastics, paper and/ or paints against microbiological contamination or growth, wherein said method comprises adding to the product 1,4-di(benzoxazol-2'-yl)benzene in an amount of 0.1 to 20 wt.-%, more preferably in an amount of 0.25 to 15 wt.-%, most preferably in an amount of 0.3 to 10 wt.-%, such as in an amount of 0.3 to 7.5 wt.-%. Further suitable ranges encompass 0.25 to 10 wt.-%, 0.25 to 7.5 wt.-%, 0.25 to 5 wt.-%, 1 to 7.5 wt.-% as well as 1 to 5 wt.-%.

In a particular advantageous embodiment, the invention relates to a method of preventing microbial decay and breakdown of cosmetic or pharmaceutical compositions furthermore comprising water and at least one further agent selected from the group consisting of surfactants, emulsifiers, thickeners, and oils as such compositions are particular sensitive to microbial growth.

The present invention furthermore relates to the use of 1,4-di(benzoxazol-2'-yl)benzene as deodorant compound as 1,4-di(benzoxazol-2'-yl)benzene has an antimicrobial action against the bacteria which are responsible for the decomposition of sweat and thus for the formation of the odor.

Advantageously, 1,4-di(benzoxazol-2'-yl)benzene can also be used in admixture with traditional preservatives to improve the preservative activity thereof.

A particular suitable mixture is the combination of 1,4-di(benzoxazol-2'-yl)benzene with at least one compound selected from the group of hydroxyacetophenone, an alkanediol and benzoic acid or a salt thereof, as 1,4-di(benzoxazol-2'-yl)benzene synergistically enhances the antimicrobial activity of said compounds. Said mixtures are still novel. Thus, the present invention also relates to a mixture comprising 1,4-di(benzoxazol-2'-yl)benzene and at least one compound selected from the group of hydroxyacetophenone, an alkanediol and benzoic acid or a salt thereof.

Preferably, said mixture is used as an antimicrobial agent mixture, which can be added to any product as defined herein to improve its resistance against microorganisms. It is well understood, that the mixture can be added as a pre-mix to the respective product, alternatively, however, the 1,4-di(benzoxazol-2'-yl)benzene and at least one compound selected from the group of hydroxyacetophenone, an alkanediol and benzoic acid or a salt thereof can also be added separately to the respective product.

It is particularly preferred that said mixture or a product comprising said mixture does not comprise a hyperbranched copolymer, such as in particular a hyperbranched of the monomers dodecenyl succinic acid anhydride, diisopropanol amine, bis-dimethylaminopropyl amine having terminal groups of formula

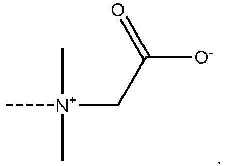

The weight ratio of 1,4-di(benzoxazol-2'-yl)benzene to the at least one compound selected from the group of hydroxyacetophenone, an alkanediol and benzoic acid or a salt thereof is preferably selected in the range of between 40:1 to 1:40, more preferred between 30:1 to 1:30, even more preferred between 20:1 and 1:20 such as in the range of 15:1 to 1:15 or 15:1 to 1:10.

Most preferably, in case of hydroxyacetophenone, the weight-ratio is selected in the range of between 30:1 to 1:2, such as between 25:1 to 1:1.

Most preferably, in case of an alkanediol the weight-ratio is selected in the range of between 15 to 1 to 1 to 5, such as between 10:1 to 1 to 1:3.

Most preferably, in case of benzoic acid or a salt thereof the weight-ratio is selected in the range of between 30:1 to 1:1, more preferably between 25:1 to 1:1.

In all embodiments, preferably, the weight-ratio of 1,4-di(benzoxazol-2'-yl)benzene to the at least one compound selected from the group of hydroxyacetophenone, an alkanediol and benzoic acid or a salt thereof is ≥ 0.5, more preferably ≥ 0.75, most preferably ≥ 1.

The term hydroxyacetophenone refers to o-, m- or p-hydroxyacetophenone. Particularly preferred in all embodiments of the present invention is p-hydroxyacetophenone [CAS 99-93-4] which is also called 1-(4-hydroxyphenyl)-ethanone and which is e.g. commercially available at Symrise as SymSave^{®} H.

In all embodiments of the present invention, the amount of hydroxyacetophenone used is selected in the range of 0.001 to 5 wt.-%, more preferably in the range of 0.01 to 4 wt-%, most preferably in the range of 0.1 to 3 wt-%, based on the total weight of the product. Further preferred ranges are 0.005 to 4.5 wt-%, 0.05 to 4 wt-%, and 0.25 to 3 wt-%, based on the total weight of the product.

Alkane diols such as in particular straight chain and branched C₁₋₁₀alkane diols are well known preservation boosters for cosmetic applications. Particularly suitable alkane diols according to the present invention encompass straight chain 1,2-, 1,3-respectively 1,4-alkane diols which comprise from about 2 to about 10 carbon atoms, e.g., from about 3 to about 8 carbon atoms, or from about 3 to about 7 carbon atoms, such as in particular 1,2-propane diol, 1,3-propanediol, 1,4-butanediol, 1,2-pentanediol, 1,2-hexanediol, 1,2-octanediol and 1,2-decanediol. The most preferred alkane diol in all embodiments of the present invention is 1,3-propandiol [CAS 504-63-2] which is e.g. commercially available under the tradename Zemea^{™} Propandiol at DuPont Tate & Lyle Bioproducts.

In all embodiments of the present invention, the amount of the alkane diol used is preferably selected in the range of 0.01 to 6 wt.-%, more preferably in the range of 0.1 to 5 wt-%, most preferably in the range of 0.5 to 4 wt-%, based on the total weight of the product. Further preferred ranges are 0.5 to 3.5 wt-% and 0.5 to 3 wt.-%, based on the total weight of the product.

Benzoic acid as well as salts thereof are well known preservatives commonly used in foods, pharmaceuticals and cosmetics. Preferred salts are the potassium or sodium salts, most preferably the sodium salt. Particularly preferred in all embodiments of the present invention is the use of benzoic acid [CAS 65-85-0] and/ or sodium benzoate [CAS 532-32-1], most preferably of sodium benzoate which is e.g. commercially available at Emerald Kalama Chemicals under the trade name Purox^{®} S Grains.

In all embodiments of the present invention, the amount of the benzoic acid or a salt thereof used is selected in the range of 0.001 to 6 wt.-%, more preferably in the range of 0.01 to 4 wt-%, most preferably in the range of 0.1 to 3 wt-%, based on the total weight of the product. Further preferred ranges encompass 0.1 to 1 wt-% and 0.1 to 0.8 wt.-%, based on the total weight of the product.

The use according to the invention of 1,4-di(benzoxazol-2'-yl)benzene, optionally in combination with at least one compound selected from the group of hydroxyacetophenone, an alkane diol and benzoic acid or a salt thereof can take place both in the cosmetic sense and in the pharmaceutical sense. In all embodiments of the present invention, the use is however preferably cosmetic (non-therapeutic).

The cosmetic or pharmaceutical compositions according to the present invention are in particular compositions intended to be topically applied to mammalian keratinous tissue such as in particular to human skin or the human scalp and hair.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Compositions", Verlag für chemische Industrie (ed. H. Ziolkowsky), 4th edition, 1992.

Suitable surfactants, emulsifiers, thickeners, and oils for the purpose of the present inventions are all surfactants, emulsifiers, thickeners, and oils commonly used in cosmetic applications and which are e.g. listed in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The compositions according to the present invention are generally prepared by admixing 1,4-di(benzoxazol-2'-yl)benzene, preferably in the form of an aqueous dispersion as defined herein in an amount (based on 1,4-di(benzoxazol-2'-yl)benzene ) selected in the range 0.1 to 20 wt.-%, more preferably in the range from about 0.25 to 15 wt.-%, most preferably in the range from about 0.3 to 10 wt.-% such as in the range from 0.3 to 7.5 wt.-%, based on the total weight of the composition with a suitable carrier. Further suitable ranges encompass 0.25 to 10 wt.-%, 0.25 to 7.5 wt.-%, 0.25 to 5 wt.-%, 1 to 7.5 wt.-% as well as 1 to 5 wt.-%.

The cosmetic or pharmaceutical compositions according to the present invention preferably further comprise a physiologically acceptable medium, that is to say a medium compatible with keratinous substances, such as the skin, mucosa, and keratinous fibres. In particular the physiologically acceptable medium is a cosmetically or pharmaceutically acceptable carrier.

The term cosmetically or pharmaceutically acceptable carrier refers to all carriers and/or excipients and/ or diluents conventionally used in cosmetic compositions or pharmaceutical compositions.

Preferably, the cosmetic or pharmaceutical compositions according to the invention are in the form of a suspension or dispersion in solvents or fatty substances, or alternatively in the form of an emulsion or micro emulsion (in particular of O/W- or W/O-type), PIT-emulsion, nano emulsion, multiple emulsion (e. g. O/W/O- or W/O/W-type), pickering emulsion, hydrogel, lipogel, one- or multiphase solution or vesicular dispersion.

The cosmetic or pharmaceutical compositions in accordance with the invention can be in the form of a liquid, lotion, a thickened lotion, a gel, a cream, a milk, an ointment or a paste.

The cosmetic or pharmaceutical compositions according to the invention generally have a pH in the range from 3-10, preferably in the range from pH of 3-8, most preferred in the range from pH 3.5-7.5. The pH is adjusted by methods known to a person skilled in the art, e.g. by using an acid such as a hydroxy acid including glycolic acid, lactic acid, malic acid, citric acid and tartaric acid or a base such as e.g. sodium or potassium hydroxide or ammonium hydroxide as well as mixtures thereof.

Preferably, in the compositions according to the invention the acid or the base, if present, is used in an amount of at least 0.0001 wt.-%, such as e.g. in an amount of 0.01-1 wt.-%, in particular in an amount of 0.01 to 0.5 wt.-%.

The cosmetic compositions according to the present invention are in particular skin care preparations, functional preparations and/ or hair care preparations such as most in particularly skin or hair care preparations.

Examples of skin care preparations are, in particular, light protective preparations (sun care preparations), anti-ageing preparations, preparations for the treatment of photo-ageing, body oils, body lotions, body gels, treatment creams, skin protection ointments, moisturizing preparations such as moisturizing gels or moisturizing sprays, face and/or body moisturizers, as well as skin lightening preparations.

Examples of functional preparations are cosmetic compositions containing active ingredients such as hormone preparations, vitamin preparations, vegetable extract preparations, anti-ageing preparations, and/or antimicrobial (antibacterial or antifungal) preparations without being limited thereto.

Examples of hair care preparations which are suitable according to the invention and which may be mentioned are shampoos, hair conditioners (also referred to as hair rinses), hairdressing compositions, hair tonics, hair regenerating compositions, hair lotions, water wave lotions, hair sprays, hair creams, hair gels, hair oils, hair pomades or hair brilliantines. Accordingly, these are always preparations which are applied to the hair and the scalp for a shorter or longer time depending on the actual purpose for which they are used.

Preferably in all embodiments of the present invention the skin care preparation is a sun care preparation.

It is preferred that said sun care preparation comprises at least one UV filter. The UV filter may be a liquid or solid UV filter. The UV filter may be a UV-A or UV-B filters. Suitable liquid UV-filter absorb light in the UVB (280 - 315 nm) and/ or UVA (315 - 400 nm) range and are liquid at ambient temperature (i.e. 25°C). Such liquid UV-filter are well known to a person in the art and encompass in particular cinnamates such as e.g. octyl methoxycinnamate (PARSOL^{®} MCX) and isoamyl methoxycinnamate (Neo Heliopan^{®} E 1000), salicylates such as e.g. homosalate (3,3,5 trimethylcyclohexyl 2-hydroxybenzoate, PARSOL^{®} HMS) and ethylhexyl salicylate (also known as ethylhexyl salicylate, 2 ethylhexyl 2-hydroxybenzoate, PARSOL^{®} EHS), acrylates such as e.g. octocrylene (2 ethylhexyl 2-cyano-3,3-diphenylacrylate, PARSOL^{®} 340) and ethyl 2-cyano-3,3 diphenylacrylate, esters of benzalmalonic acid such as in particular dialkyl benzalmalonates such as e.g. di (2-ethylhexyl) 4-methoxy¬benz¬al¬malonate and polysilicone 15 (PARSOL^{®} SLX), dialkylester of naphthalates such as e.g. diethylhexyl 2,6-naphthalate (Corapan^{®} TQ), syringylidene malo¬na¬tes such as e.g. diethylhexyl syringylidene malonate (Oxynex^{®} ST liquid) as well as benzotriazolyl dodecyl p-cresol (Tinoguard^{®} TL) as well as benzophenone-3 and drometrizole trisiloxane.

Particular advantageous liquid UV-filter are octyl methoxycinnamate, homosalate, ethylhexyl salicylate, octocrylene, diethylhexyl 2,6-naphthalate, diethylhexyl syringylidene malonate, benzotriazolyl dodecyl p-cresol, benzo-phenone-3, drometrizole trisiloxane, Polysilicone-15 as well as mixtures thereof.

Suitable solid UV-filter absorb light in the UVB and/ or UVA range and are solid at ambient temperature (i.e. 25°C). They are particularly solid organic UV filters. Particularly suited solid UV-filters are of the group consisting of bis-ethylhexyl-oxyphenol methoxyphenyl triazine, butyl methoxydibenzoyl methane, methylene bis-benzotriazolyl tetramethylbutylphenol, diethylamino hydroxybenzoyl hexyl benzoate, ethylhexyl triazone, diethylhexyl butamido triazone, and 4-methyl¬benzyli¬dene camphor.

The amount of an individual organic UV filter is preferably in the range of 0.1 to about 10 % by weight, preferable in the range of 0.5 to 7.55 % by weight, most preferably in the range of 1 to 5 % by weight, based on the total weight of the sun care preparation.

In case of a sun care composition, the total amount of organic UV filter (s) depends strongly on the targeted UV protection of said composition and is typically in the range of between 1 to 50% by weight, preferably between 5 to 40% by weight, based on the total weight of said sun care preparation.

A sun care preparation with an SPF 15 (SPF=sun protection factor), for example, comprises preferably a total amount of organic UV filter (s) of between 4 to 20% by weight, more preferably between 7 and 15 % by weight, based on the total weight of said sun care preparation.

A sun care preparation with an SPF 30, for example, comprises preferably a total amount of organic UV filter (s) of between 10 to 40% by weight, more preferably between 15 and 25 % by weight, based on the total weight of said sun care preparation.

A sun care preparation with an SPF 50, for example, comprises preferably a total amount of organic UV filter (s) of between 15 to 50% by weight, more preferably between 20 and 40 % by weight, based on the total weight of said sun care preparation.

In a preferred embodiment, the cosmetic compositions according to the present invention are emulsions and/ or gels. Even more preferably, the cosmetic compositions are emulsions which contain an oily phase and an aqueous phase such as in particular O/W, W/O, Si/W, W/Si, O/W/O, W/O/W multiple or a pickering emulsions.

The amount of the oily phase (i.e. the phase containing all oils and fats including the polar oils) present in such emulsions is preferably at least 10 wt.-%, such as in the range from 10 to 60 wt.-%, preferably in the range from 15 to 50 wt.-%, most preferably in the range from 15 to 40 wt.-%, based on the total weight of the topical composition.

The amount of the aqueous phase present in such emulsions is preferably at least 20 wt.-%, such as in the range from 20 to 90 wt.-%, preferably in the range from 30 to 80 wt.-%, most preferably in the range from 30 to 70 wt.-%, based on the total weight of the topical composition.

The O/W emulsions according to the present invention advantageously comprise (i) 1,4-di(benzoxazol-2'-yl)benzene in an amount selected in the range from 0.1 to 20 wt. %, more preferably in the range from about 0.25 to 15 wt. %, most preferably in the range from about 0.3 to 10 wt.-% such as in the range from 0.3 to 7.5 wt. %, based on the total weight of the composition, (ii) water and (iii) at least one O/W- or Si/W-emulsifier selected from the list of glycerylstearatcitrate, glycerylstearate (self-emulsifying), stearic acid, salts of stearic acid, polyglyceryl-3-methylglycosedistearate, ceteareth-20, steareth-2, steareth-12, PEG-40 stearate, phosphate esters and the salts thereof such as cetyl phosphate (Amphisol^{®} A), diethanolamine cetyl phosphate (Amphisol^{®} DEA), potassium cetyl phosphate (Amphisol^{®} K), sodiumcetearylsulfat, sodium glyceryl oleate phosphate, hydrogenated vegetable glycerides phosphate, sorbitan oleate, sorbitan sesquioleate, sorbitan isostearate, sorbitan trioleate, lauryl glucoside, decyl glucoside, sodium stearoyl glutamate, sucrose polystearate and Hydrated Polyisobuten as well as mixtures thereof. Also, one or more synthetic polymers may be used as an emulsifier such as for example, PVP eicosene copolymer, acrylates/C10-3o alkyl acrylate crosspolymer, acrylates/steareth-20 methacrylate copolymer, PEG-22/dodecyl glycol copolymer, PEG-45/dodecyl glycol copolymer, and mixtures thereof. In a particular preferred embodiment the O/W-emulsifier is selected from the group of cetyl phosphates such as in particular potassium cetyl phosphate (commercially available as Amphisol^{®} K), glyceryl stearate (and) PEG-100 stearate (commercially available as Arlacel^{®} 165), Polyacrylamide, Sodium Polyacrylate, Sodium Polyacryloyldimethyl Taurate, Decyl Glucoside, Ceteral Glucoside, Caprylyl/Capryl Glucoside, Sorbitan Olivate, Polygelyceryl-4 Olivate, Polyglyceryl-3 Methylglucose Distearate, Polysorbate 20, Polysorbate 60 and/ or polyalkylenglycolether such as in particular laureth-35 (lauryl alcohol with 35 EO units; commercially available as Brij^{®} 35). The at least one O/W emulsifier is preferably used in an amount of about 0.001 to 10 wt.-%, more preferably in an amount of 0.1 to 7 wt.-% with respect to the total weigh of the composition. Additionally, the cosmetic composition in the form of a O/W emulsion contains advantageously at least one co-emulsifier selected from the list of alkyl alcohols such as Cetyl Alcohol (Lorol C16, Lanette 16) Cetearyl Alcohol (Lanette^{®} O), Stearyl Alcohol (Lanette^{®} 18), Behenyl Alcohol (Lanette^{®} 22), Glyceryl Monostearate, Glyceryl Myristate (Estol^{®} 3650), Hydrogenated Coco- Glycerides (Lipocire Na10) without being limited to this and mixtures thereof.

The W/O emulsions according to the present invention advantageously comprise (i) 1,4-di(benzoxazol-2'-yl)benzene in an amount selected in the range from 0.1 to 20 wt. %, more preferably in the range from about 0.25 to 15 wt. %, most preferably in the range from about 0.3 to 10 wt.-% such as in the range from 0.3 to 7.5 wt. %, based on the total weight of the composition, (ii) water and (iii) at least one W/O- or W/Si-emulsifier selected from the list of polyglyceryl-2-dipolyhydroxystearat, PEG-30 dipolyhydroxystearat, cetyl dimethicone copolyol, polyglyceryl-3 diisostearate polyglycerol esters of oleic/isostearic acid, polyglyceryl-6 hexaricinolate, polyglyceryl-4-oleate, polygylceryl-4 oleate/PEG-8 propylene glycol cocoate, magnesium stearate, sodium stearate, potassium laurate, potassium ricinoleate, sodium cocoate, sodium tallowate, potassium castorate, sodium oleate, and mixtures thereof. Further suitable W/Si-emulsifiers are Lauryl Polyglyceryl-3 Polydimethylsiloxyethyl Dimethicone and/or PEG-9 Polydimethylsiloxyethyl Dimethicone and/or Cetyl PEG/PPG-10/1 Dimethicone and/or PEG-12 Dimethicone Crosspolymer and/or PEG/PPG-18/18 Dimethicone. The at least one W/O emulsifier is preferably used in an amount of about 0.001 to 10 wt.-%, more preferably in an amount of 0.2 to 7 wt.-% with respect to the total weigh of the composition.

The gel preparations according to the present invention advantageously comprise (i) 1,4-di(benzoxazol-2'-yl)benzene in an amount selected in the range from 0.1 to 20 wt. %, more preferably in the range from about 0.25 to 15 wt. %, most preferably in the range from about 0.3 to 10 wt.-% such as in the range from 0.3 to 7.5 wt. %, based on the total weight of the composition, (ii) water and (iii) at least one water soluble thickener.

Such water-soluble thickeners are well known to a person skilled in the art and are e.g. listed in the "Handbook of Water soluble gums and resins" by Robert L. Davidson (Mc Graw Hill Book Company (1980)). Particularly suitable water soluble thickeners are selected from the group consisting of polyacrylic acids (e.g. commercially available under the tradename Carbomer or Carbopol^{®}), homopolymers of 2-Acrylamido-2-methylpropansulfonic acid (e.g. commercially available as Rheothik^{®}11-80), acrylate copolymers (e.g. commercially available under the tradename Pemulen^{®} or Aculyn^{®} 33), branched Poly(methacryloyloxyethyltrimethylammoniumchlorid) (INCI-name Polyquaternium-37), non-modified guar gums (e.g. commercially available under the tradename Jaguar), starch or derivatives thereof and/ or hydroxyalkylcelluloses. Preferably the water-soluble thickener is used in an amount of about 0.001 to 10 wt.-%, more preferably in an amount of 0.1 wt.-% to 7 wt.-%, based on the total weigh of the composition.

The following examples are provided to further illustrate the compositions and effects of the present invention. These examples are illustrative only and are not intended to limit the scope of the invention in any way.

### Examples

### 1. Preparation of 1,4-di(benzoxazol-2'-yl)benzene:

A mixture of 702 g polyphosphoric acid and 4.28 ml methanesulfonic acid was heated to 90°C. 65 g terephthalic acid and 107 g 2-aminophenol were added. The mixture was stirred under inert atmosphere at 180°C for 8 hours and then transferred to ice water. The precipitated product was filtered and washed with water and acetic acid. The precipitate was dispersed in water and the pH adjusted to 8.0 with sodium hydroxide, filtered and washed with water. The crude product was suspended in a mixture of toluene and 1-butanol 3.3:1, stirred at 85°C for one hour, filtered, washed with diethyl ether, and dried resulting in coarse particles of solid amorphous 1,4-di(benzoxazol-2'-yl)benzene (in the following referred to as DBO).

### 2. Preparation of 1,4-di(benzoxazol-2'-yl)benzene, 30wt.-% aqueous dispersion:

Afterwards, a suspension of 175 g of the coarse particles, 324 g of water and 65 g Green APG 0810 was prepared and the suspension was milled for 2h with a LabStar laboratory mill using yttrium-stabilized zirconium oxide grinding beads (0.3mm, from Tosoh Ceramic, Japan) and cooling of the milling chamber (-12°C brine). After removal of the grinding beads, a 30% aqueous dispersion of micronized 1,4-di(benzoxazol-2'-yl)benzene (in the following referred to as DBO aq.) was obtained which was used in the antimicrobial tests and formulations as outlined below.

### 3. Antimicrobial activity

The antimicrobial efficacy was assessed in analogy to the regulatory challenge test method (NF EN ISO11930). Thus, mixtures of the respective active(s) with physiological serum with 0.85 wt.-% NaCl in the concentrations as outlined below are prepared under sterile conditions. Controls were also prepared under sterile conditions. The control as well as the mixtures were then deposed in 96-deep well plates (1.6 ml/well). The wells were contaminated with the respective bacterial or the fungal strains at 2.5_{*}10⁵ to 5.6_{*}10⁵ cfu/ml for the bacteria and 1_{*}10⁴ to 2.5_{*}10⁴ cfu/ml for the fungi to obtain the initial contamination as outlined below. After the contamination, each well was thoroughly mixed to ensure a homogeneous distribution of microbes. Then each plate was incubated at 22°C for 24h. The counting of the (remaining) population is carried out 24h after contamination. Then, the average log reduction is calculated [-log10{(value inoculum)/ (value sample)}] which is represented in tables 1-5.

**Table 1: Results using 0.3 wt.-% DBO**

| Microbe | inoculum | 24h | Log reduction |
|---|---|---|---|
| | colony count [cfu/ml] | | |
| Candida albicans (preser., yeast) | 32000 | 10000 | -0.51 |
| Aspergillus brasiliensis (preserv., mold) | 45000 | 400 | -2.05 |

**Table 2: Results using 1 wt.-% DBO aq. (0.3 wt.-% active)**

| Microbe | inoculum | 24h | Log reduction |
|---|---|---|---|
| | colony count [cfu/ml] | | |
| Candida albicans (preser., yeast) | 70000 | 40000 | -0.24 |
| Escherichia coli (preserv., gram-) | 400000 | 10000 | -1.60 |
| Staphylococcus aureus (preserv., deo, gram+) | 550000 | 100000 | -0.74 |
| Pseudomonas aeruginosa (preserv., gram-) | 330000 | 1000 | -2.52 |
| Aspergillus brasiliensis (preserv., mold) | 40000 | 1000 | -1.60 |

As can be seen in the tables above 1,4-di(benzoxazol-2'-yl)benzene has an antimicrobial effect over a broad range of microbes and can thus be used as preservation booster. Since it is known that typically the water phase of a product is most susceptible to microbial growth, 1,4-di(benzoxazol-2'-yl)benzene is thus particularly suitable to protect products comprising a water phase such as e.g. cosmetic compositions in the form of an O/W or W/O emulsion against microbial decay and breakdown and is thus suitable for improving its preservation.

**Table 3: Synergistic effect with p-Hydroxyacetophenone (HAP)**

| Test solution | Time [h] | Staphylococcus aureus colony count [cfu/ml] | Log reduction |
|---|---|---|---|
| 1.0 wt.-% DBO aq.* | 0 | 550000 | |
| | 24 | 100000 | -0.74 |
| 0.3 wt.-% HAP | 0 | 550000 | |
| | 24 | 70000 | -1.16 |
| 1.0 wt.-% DBO aq.* | 0 | 550000 | |
| 0.3 wt.-% HAP | 24 | 100 | -3.74 |
| Control | 0 | 550000 | |
| | 24 | 1000000 | +0.26 |

| | | | |
|---|---|---|---|
| *0.3 wt.-% active | | | |

As can be retrieved from table 2 DBO synergistically enhances the antimicrobial activity of p-Hydroxyacetophenone.

**Table 4: Synergistic effect with sodium benzoate**

| Test solution | Time [h] | Escherichia coli colony count [cfu/ml] | Log reduction |
|---|---|---|---|
| 1.0 wt.-% DBO aq.* | 0 | 400000 | |
| | 24 | 10000 | -1.60 |
| 0.2 wt.-% Sodium Benzoate | 0 | 400000 | |
| | 24 | 1000000 | +0.40 |
| 1.0 wt.-% DBO aq.* | 0 | 400000 | |
| 0.2 wt.-% Sodium Benzoate | 24 | 100 | -3.60 |
| Control | 0 | 400000 | |
| | 24 | 1000000 | +0.40 |

| | | | |
|---|---|---|---|
| *0.3 wt.-% active | | | |

As can be retrieved from table 3 the combination of DBO and sodium benzoate exhibits a synergistic antimicrobial activity.

**Table 5: Synergistic effect with 1,3-propanediol (PDO)**

| Test solution | Time [h] | Candida albicans colony count [cfu/ml] | Log reduction |
|---|---|---|---|
| 1.0 wt.-% DBO aq.* | 0 | 70000 | |
| | 24 | 40000 | -0.24 |
| 1.0 wt.-% PDO | 0 | 70000 | |
| | 24 | 40000 | -0.24 |
| 1.0 wt.-% DBO aq.3 | 0 | 70000 | |
| 1.0 wt.-% PDO | 24 | 10000 | -0.85 |
| Control | 0 | 70000 | |
| | 24 | 70000 | 0 |

| | | | |
|---|---|---|---|
| *0.3 wt.-% active | | | |

As can be retrieved from table 4 the combination of DBO and 1,3-propanediol exhibits a synergistic antimicrobial activity.

### 4. Formulation examples

### Sunscreen o/w

| Phase | INCI | Weight-% | | |
|---|---|---|---|---|
| A | PHENYLBENZIMIDAZOLE SULFONIC ACID | 0.5 | 0.5 | 0.5 |
| | AQUA | 10 | 10 | 10 |
| | TRIETHANOLAMINE | 0.4 | 0.4 | 0.4 |
| B | DISODIUM EDTA; AQUA | 0.1 | 0.1 | 0.1 |
| | AQUA | Add 100 | | |
| | BUTYLENE GLYCOL | 4 | 4 | 4 |
| | ACACIA SENEGAL GUM; XANTHAN GUM | 0.3 | 0.3 | 0.3 |
| | COCO-GLUCOSIDE; AQUA | 0.5 | 0.5 | 0.5 |
| | PROPANEDIOL | | | 3 |
| | SODIUM BENZOATE | 0.4 | | |
| C | C15-19 ALKANE | 5 | 5 | 5 |
| | OCTOCRYLENE | 6 | 6 | 6 |
| | POTASSIUM CETYL PHOSPHATE | 1.8 | 1.8 | 1.8 |
| | TOCOPHERYL ACETATE | 0.5 | 0.5 | 0.5 |
| | METHOXYDIBENZOYLMETHANE | 2.5 | 2.5 | 2.5 |
| | ETHYLHEXYL SALICYLATE | 5 | 5 | 5 |
| | TITANIUM DIOXIDE; SILICA; DIMETHICONE | 3 | 3 | 3 |
| | PHENOXYETHANOL; ETHYLHEXYLGLYCERIN | 1 | 1 | 1 |
| | DIISOPROPYL ADIPATE; PROPYLENE GLYCOL DICAPRYLATE/DICAPRATE; C12-15 ALKYL BENZOATE; DIISOPROPYL SEBACATE | 3 | 3 | 3 |
| D | SILICA | 1 | 1 | 1 |
| | DBO aq. | 1-3 | 1-3 | 1-3 |
| E | HYDROXYETHYL ACRYLATE/SODIUM ACRYLOYLDIMETHYL TAURATE COPOLYMER; | 1 | 1 | 1 |
| | POLYISOBUTENE; PEG-7 TRIMETHYLOLPROPANE COCONUT ETHER | | | |
| | HYDROXYACETOPHENONE | | 0.75 | 0.6 |
| F | AQUA; SODIUM HYDROXIDE | 1.45 | 1.45 | 1.45 |

### Crème Gel

| Phase | INCI | Weight.-% | | |
|---|---|---|---|---|
| A | AQUA | Add 100 | | |
| | GLYCERIN | 3 | 3 | 3 |
| | PHENOXYETHANOL; ETHYLHEXYLGLYCERIN | 1 | | |
| | PROPANEDIOL | | | 3 |
| | SODIUM BENZOATE | | 0.5 | |
| B | CAPRYLIC/CAPRIC TRIGLYCERIDE | 5 | 5 | |
| | ETHYLHEXYL OLIVATE; SODIUM ACRYLATES COPOLYMER; POLYGLYCERYL-4 OLIVATE | 1.5 | 1.5 | |
| C | DBO aq. | 1-3 | 1-3 | 1-3 |
| | HYDROXYACETOPHENONE | 0.1 | | 0.7 |
| | SODIUM HYDROXIDE; AQUA | 0.05 | | 0.05 |
| | CITRIC ACID; AQUA | | 0.1 | |

### Sunscreen w/o

| Phase | INCI | Weight-% | | |
|---|---|---|---|---|
| A | AQUA | Add 100 | | |
| | PROPANEDIOL | 3.0 | | 3.0 |
| B | LAURYL PEG-10 TRIS(TRIMETHYLSILOXY)SILYLETHYL DIMETHICONE | 6.0 | 6.0 | 6.0 |
| | OCTOCRYLENE | 3.0 | 3.0 | 3.0 |
| | BUTYL METHOXYDIBENZOYLMETHANE | 2.0 | 2.0 | 2.0 |
| | POLYSILICONE-15 | 3.0 | 3.0 | 3.0 |
| | ZINC OXIDE; TRIETHOXYCAPRYLYLSILANE | 11.7 | 11.7 | 11.7 |
| | HOMOSALATE | 7.5 | 7.5 | 7.5 |
| | TITANIUM DIOXIDE; SILICA; DIMETHICONE | 3.0 | 3.0 | 3.0 |
| | DIMETHICONE | 3.0 | 3.0 | 3.0 |
| | ISONONYL ISONONANOATE | 10.0 | 10.0 | 10.0 |
| C | CYCLOPENTASILOXANE | 20.0 | 20.0 | 20.0 |
| | ALCOHOL DENAT. | 5.0 | 5.0 | 5.0 |
| | DBO aq. | 1-3 | 1-3 | 1-3 |
| | SODIUM BENZOATE | | 0.5 | |
| | HYDROXYACETOPHENONE | | | 0.2 |

### Skin Care o/w

| Phase | INCI | Weight-% | | |
|---|---|---|---|---|
| A | AQUA | Add 100 | | |
| | PROPANEDIOL | | | |
| | DISODIUM EDTA; AQUA | 0.05 | 0.05 | 0.05 |
| | XANTHAN GUM | 0.2 | 0.2 | 0.2 |
| | ETHOXYDIGLYCOL | 2.0 | 2.0 | 2.0 |
| | CETEARYL OLIVATE; SORBITAN OLIVATE | 4.0 | 4.0 | 4.0 |
| | DIMETHICONE | 2.0 | 2.0 | 2.0 |
| B | ISOPROPYL MYRISTATE | 4.0 | 4.0 | 4.0 |
| | DICAPRYLYL ETHER | 5.0 | 5.0 | 5.0 |
| | PARAFFINUM LIQUIDUM | 2.0 | 2.0 | 2.0 |
| | STEARIC ACID; PALMITIC ACID | 1.5 | 1.5 | 1.5 |
| | ISOSTEARYL ISOSTEARATE | 5.0 | 5.0 | 5.0 |
| C | DBO aq. | 1-3 | 1-3 | 1-3 |
| | SODIUM BENZOATE | 0.6 | | 4.0 |
| | HYDROXYACETOPHENONE | | 0.75 | 0.5 |
| | SODIUM HYDROXIDE; AQUA | 0.08 | 0.08 | 0.08 |

### Skin Care o/w

| Phase | INCI | Weight-% | | |
|---|---|---|---|---|
| A | AQUA | Add 100 | | |
| | HYDROXYPROPYL STARCH PHOSPHATE; AQUA | 1.0 | 1.0 | 1.0 |
| | HYDROXYACETOPHENONE | 0.6 | 0.5 | |
| | PROPANEDIOL | | 3.0 | |
| | SODIUM BENZOATE | | | 0.5 |
| | GLYCERIN | 2.0 | 2.0 | 2.0 |
| B | ISOPROPYL MYRISTATE | 2.0 | 2.0 | 2.0 |
| | CETEARYL ALCOHOL | 2.0 | 2.0 | 2.0 |
| | SQUALANE | 2.0 | 2.0 | 2.0 |
| | POTASSIUM CETYL PHOSPHATE | 3.0 | 3.0 | 3.0 |
| | COCOGLYCERIDES | 5.5 | 5.5 | 5.5 |
| | C12-15 ALKYL BENZOATE | 4.0 | 4.0 | 4.0 |
| | HYDROGENATED COCOGLYCERIDES | 4.0 | 4.0 | 4.0 |
| | PARAFFINUM LIQUIDUM | 3.0 | 3.0 | 3.0 |
| C | TOCOPHEROL | 0.2 | 0.2 | 0.2 |
| | SACCHARIDE ISOMERATE; AQUA; CITRIC ACID; SODIUM CITRATE | 1.0 | 1.0 | 1.0 |
| | ARGANIA SPINOSA KERNEL OIL | 2.0 | 2.0 | 2.0 |
| | DBO aq. | 1-3 | 1-3 | 1-3 |

### CC Cream o/w

| Phase | INCI | Weight-% | | |
|---|---|---|---|---|
| A | AQUA | Add 100 | | |
| | PROPANEDIOL | | 4.0 | 4.0 |
| | HYDROXYACETOPHENONE | 0.7 | 0.5 | |
| | SODIUM BENZOATE | | | 0.4 |
| B | MICROCRISTALLINE CELLULOSE ; CELLULOSE GUM | 2.0 | 2.0 | 2.0 |
| C | ETHYLHEXYL METHOXYCINNAMATE | 10.0 | 10.0 | 10.0 |
| | BEHENYL ALCOHOL | 4.0 | 4.0 | 4.0 |
| | GLYCERYL MYRISTATE | 4.0 | 4.0 | 4.0 |
| | ISOAMYL LAURATE | 14.0 | 14.0 | 14.0 |
| | OCTYLDODECANOL | 8.0 | 8.0 | 8.0 |
| | DIMETHICONE | 4.0 | 4.0 | 4.0 |
| | TITANIUM DIOXIDE; AQUA; GLYCERIN; PHENOXYETHANOL; CELLULOSE GUM; SODIUM POLYACRYLATE | 8.0 | 8.0 | 8.0 |
| | CI77499; AQUA; GLYCERIN; PHENOXYETHANOL; CELLULOSE GUM; SODIUM POLYACRYLATE | 0.25 | 0.25 | 0.25 |
| | CI77491; AQUA; GLYCERIN; PHENOXYETHANOL; CELLULOSE GUM; SODIUM POLYACRYLATE | 0.545 | 0.545 | 0.545 |
| | CI77492; AQUA; GLYCERIN; PHENOXYETHANOL; CELLULOSE GUM; SODIUM POLYACRYLATE | 2.2 | 2.2 | 2.2 |
| | TITANIUM DIOXIDE; SILICA; DIMETHICONE | 3.0 | 3.0 | 3.0 |
| | POTASSIUM CETYL PHOSPHATE | 3.0 | 3.0 | 3.0 |
| D | DBO aq. | 1-3 | 1-3 | 1-3 |

## Claims

1. non-therapeutic Use of 1,4-di(benzoxazol-2'-yl)benzene as antimicrobial agent.

2. The use according to claim 1, wherein the antimicrobial agent is an antifungal and/ or antibacterial agent.

3. The use according to claim 2, wherein the antifungal and/ or antibacterial agent is an agent that inhibits the growth of Candida albicans, Escherichia coli, Staphylococcus aureus, Pseudomonas aeruginosa and/ or Aspergillus brasiliensis as well as mixtures thereof.

4. The use according to anyone of the preceding claims in a product selected from the group of cosmetic compositions, household products, plastics, paper and/ or paints.

5. The use according to anyone of the preceding claims for improving preservation.

6. Non-therapeutic use of 1,4-di(benzoxazol-2'-yl)benzene as deodorant active compound.

7. The use according to anyone of the preceding claims, wherein the 1,4-di(benzoxazol-2'-yl)benzene is used in micronized form.

8. The use according to anyone of the preceding claims, wherein the 1,4-di(benzoxazol-2'-yl)benzene is used in combination with at least one compound selected from the group of hydroxyacetophenone, an alkanediol and benzoic acid or a salt thereof.

9. The use according to claim 8, wherein the at least one compound is selected from the group of p-hydroxyacetophenone, benzoic acid, sodium benzoate and 1,3-propanediol.

10. A method of preventing microbial decay and breakdown of a product selected from the group of cosmetic and/ or pharmaceutical compositions, household products, plastics, paper and/ or paints, wherein said method comprises adding to the product 1,4-di(benzoxazol-2'-yl)benzene in an amount selected in the range from 0.1 to 20 wt.-%, more preferably in the range from about 0.25 to 15 wt.-%, most preferably in the range from about 0.3 to 10 wt.-% such as in the range from 0.3 to 7.5 wt.-%, based on the total weight of the product.

11. The method according to claim 10, wherein the product furthermore comprises water and at least one agent selected from the group consisting of surfactants, emulsifiers, thickeners and oils.

12. The method according to claim 10 or 11, wherein the product is a cosmetic composition in the form of an O/W emulsion, a W/O emulsion or a gel.

13. A non-therapeutic method for killing and/ or inhibiting growth of microbial cells, in particular fungal and/ or bacterial cells, said method comprising contacting said microbial cells with 1,4-di(benzoxazol-2'-yl)benzene, optionally in admixture with at least one compound selected from the group of hydroxyacetophenone, an alkanediol, benzoic acid or a salt thereof.

14. The method according to claim 13, wherein the at least one compound is present and selected from the group of p-hydroxyacetophenone, benzoic acid, sodium benzoate and 1,3-propanediol

15. The method according to claim 12, wherein the microorganism is selected from the group consisting of Candida albicans, Escherichia coli, Staphylococcus aureus, Pseudomonas aeruginosa and/ or Aspergillus brasiliensis as well as mixtures thereof.

## Patentansprüche

1. Nichttherapeutische Verwendung von 1,4-Di(benzoxazol-2'-yl)benzol als antimikrobielles Mittel.

2. Verwendung nach Anspruch 1, wobei es sich bei dem antimikrobiellen Mittel um ein antimykotisches und/oder antibakterielles Mittel handelt.

3. Verwendung nach Anspruch 2, wobei es sich bei dem antimykotischen und/oder antibakteriellen Mittel um ein Mittel handelt, das das Wachstum von Candida albicans, Escherichia coli, Staphylococcus aureus, Pseudomonas aeruginosa und/oder Aspergillus brasiliensis sowie Gemischen davon inhibiert.

4. Verwendung nach einem der vorhergehenden Ansprüche in einem Produkt, das aus der Gruppe von kosmetischen Zusammensetzungen, Haushaltsprodukten, Kunststoffen, Papier und/oder Anstrichmitteln ausgewählt ist.

5. Verwendung nach einem der vorhergehenden Ansprüche zur Verbesserung der Konservierung.

6. Nichttherapeutische Verwendung von 1,4-Di(benzoxazol-2'-yl)benzol als Deodorantwirkstoff.

7. Verwendung nach einem der vorhergehenden Ansprüche, wobei das 1,4-Di(benzoxazol-2'-yl)benzol in mikronisierter Form verwendet wird.

8. Verwendung nach einem der vorhergehenden Ansprüche, wobei das 1,4-Di(benzoxazol-2'-yl)benzol in Kombination mit mindestens einer Verbindung ausgewählt aus der Gruppe p-Hydroxyacetophenon, einem Alkandiol und Benzoesäure oder einem Salz davon verwendet wird.

9. Verwendung nach Anspruch 8, wobei die mindestens eine Verbindung aus der Gruppe p-Hydroxyacetophenon, Benzoesäure, Natriumbenzoat und 1,3-Propandiol ausgewählt ist.

10. Verfahren zur Verhinderung von mikrobiellem Verderb und Abbau eines Produkts aus der Gruppe von kosmetischen und/oder pharmazeutischen Zusammensetzungen, Haushaltsprodukten, Kunststoffen, Papier und/oder Anstrichmitteln, wobei das Verfahren das Zugeben von 1,4-Di(benzoxazol-2'-yl)benzol zu dem Produkt in einer Menge ausgewählt im Bereich von 0,1 bis 20 Gew.-%, weiter bevorzugt im Bereich von etwa 0,25 bis 15 Gew.-%, ganz besonders bevorzugt im Bereich von etwa 0,3 bis 10 Gew.-%, wie im Bereich von 0,3 bis 7,5 Gew.-%, bezogen auf das Gesamtgewicht des Produkts, umfasst.

11. Verfahren nach Anspruch 10, wobei das Produkt weiterhin Wasser und mindestens ein Mittel ausgewählt aus der Gruppe bestehend aus Tensiden, Emulgatoren, Verdickern und Ölen umfasst.

12. Verfahren nach Anspruch 10 oder 11, wobei es sich bei dem Produkt um eine kosmetische Zusammensetzung in Form einer O/W-Emulsion, einer W/O-Emulsion oder eines Gels handelt.

13. Nichttherapeutisches Verfahren zum Abtöten und/oder zur Inhibierung des Wachstums von Mikrobenzellen, insbesondere Pilz- und/oder Bakterienzellen, wobei das Verfahren das Inkontaktbringen der Mikrobenzellen mit 1,4-Di(benzoxazol-2'-yl)benzol, gegebenenfalls in Abmischung mit mindestens einer Verbindung ausgewählt aus der Gruppe Hydroxyacetophenon, einem Alkandiol und Benzoesäure oder einem Salz davon, umfasst.

14. Verfahren nach Anspruch 13, wobei die mindestens eine Verbindung vorliegt und aus der Gruppe p-Hydroxyacetophenon, Benzoesäure, Natriumbenzoat und 1,3-Propandiol ausgewählt ist.

15. Verfahren nach Anspruch 12, wobei der Mikroorganismus aus der Gruppe bestehend aus Candida albicans, Escherichia coli, Staphylococcus aureus, Pseudomonas aeruginosa und/oder Aspergillus brasiliensis sowie Gemischen davon ausgewählt ist.

## Revendications

1. Utilisation non thérapeutique de 1,4-di(benzoxazol-2'-yl)benzène en tant qu'agent antimicrobien.

2. Utilisation selon la revendication 1, l'agent antimicrobien étant un agent antifongique et/ou antibactérien.

3. Utilisation selon la revendication 2, l'agent antifongique et/ou antibactérien étant un agent qui inhibe la croissance de Candida albicans, Escherichia coli, Staphylococcus aureus, Pseudomonas aeruginosa et/ou Aspergillus brasiliensis ainsi que des mélanges correspondants.

4. Utilisation selon l'une quelconque des revendications précédentes dans un produit choisi dans le groupe constitué par des compositions cosmétiques, des produits ménagers, des matières plastiques, du papier et/ou des peintures.

5. Utilisation selon l'une quelconque des revendications précédentes pour l'amélioration de la conservation.

6. Utilisation non thérapeutique de 1,4-di(benzoxazol-2'-yl)benzène en tant que composé actif déodorant.

7. Utilisation selon l'une quelconque des revendications précédentes, le 1,4-di(benzoxazol-2'-yl)benzène étant utilisé sous forme micronisée.

8. Utilisation selon l'une quelconque des revendications précédentes, le 1,4-di(benzoxazol-2'-yl)benzène étant utilisé en combinaison avec au moins un composé choisi dans le groupe composé par une hydroxyacétophénone, un alcanediol et l'acide benzoïque ou un sel correspondant.

9. Utilisation selon la revendication 8, l'au moins un composé étant choisi dans le groupe composé par la p-hydroxyacétophénone, l'acide benzoïque, le benzoate de sodium et le 1,3-propanediol.

10. Procédé de prévention contre une décomposition et une dégradation microbienne d'un produit choisi dans le groupe composé par des compositions cosmétiques et/ou pharmaceutique, des produits ménagers, des matières plastiques, du papier et/ou des peintures, ledit procédé comprenant l'ajout, au produit, de 1,4-di(benzoxazol-2'-yl)benzène en une quantité choisie dans la plage de 0,1 à 20 % en poids, plus préférablement dans la plage d'environ 0,25 à 15 % en poids, le plus préférablement dans la plage d'environ 0,3 à 10 % en poids, tel que dans la plage de 0,3 à 7,5 % en poids, sur la base du poids total du produit.

11. Procédé selon la revendication 10, le produit comprenant en outre de l'eau et au moins un agent choisi dans le groupe constitué par des tensioactifs, des émulsifiants des épaississants et des huiles.

12. Procédé selon la revendication 10 ou 11, le produit étant une composition cosmétique sous la forme d'une émulsion H/E, d'une émulsion E/H ou d'un gel.

13. Procédé non thérapeutique pour détruire et/ou inhiber la croissance de cellules microbiennes, en particulier de cellules fongiques et/ou bactériennes, ledit procédé comprenant la mise en contact desdites cellules microbiennes avec du 1,4-di(benzoxazol-2'-yl)benzène, éventuellement en mélange avec au moins un composé choisi dans le groupe composé par par une hydroxyacétophénone, un alcanediol, l'acide benzoïque ou un sel correspondant.

14. Procédé selon la revendication 13, l'au moins un composé étant présent et étant choisi dans le groupe constitué par la p-hydroxyacétophénone, l'acide benzoïque, le benzoate de sodium et le 1,3-propanediol.

15. Procédé selon la revendication 12, le micro-organisme étant choisi dans le groupe constitué par Candida albicans, Escherichia coli, Staphylococcus aureus, Pseudomonas aeruginosa et/ou Aspergillus brasiliensis ainsi que des mélanges correspondants.
